# EUROPEAN PATENT APPLICATION

(11) **EP 2 476 374 A1**
(43) Date of publication of application: **18.07.2012**
(21) Application number: 10815396.6
(22) Date of filing: 08.09.2010
(51) Int. Cl.: A61B 5/151

(54) **DISPOSABLE BLOOD COLLECTING INSTRUMENT**

(30) Priority: 10.09.2009 JP 2009209607
(71) Applicant: NIPRO CORPORATION, Kita-ku Osaka-shi, Osaka 531-8510 (JP)
(72) Inventor: UCHIMURA Tomohiro, Osaka-shi Osaka 531-8510 (JP); ISHIKURA Kohzo, Osaka-shi Osaka 531-8510 (JP); SUZUKI Ken, Osaka-shi Osaka 531-8510 (JP)
(74) Representative: Bertsch, Florian Oliver
(86) International application number: PCT/JP2010/065446
(87) International publication number: WO 2011/030801

(57) **Abstract**

Disclosed is a disposable blood collecting instrument that stably secures a sufficient volume of blood with a single puncture. A tip portion (63) of a piercing body (62) of a lancet (11) contained within a housing (10) projects by means of a biasing force of a spring assembly (12), and a surrounding area of an opening (38) of the housing (10) is made a transparent visible portion (59) with a visible interior. Additionally, the disposable blood collecting instrument has been formed such that the spring assembly (12) is contained within housing farther behind the piercing body (62) than a position of the tip portion (63) of the piercing body (62) that has entered within the housing (10) after piercing.

## Description

### TECHNICAL FIELD

The present invention relates to a blood collecting instrument to be used for collecting a small amount of blood, especially a disposable blood collecting instrument that is disposed after a single use.

### BACKGROUND ART

There have been some needs for collecting a small amount of one's own blood for medical purposes. For example, a diabetes patient needs to collect his/her own blood to monitor its blood glucose level (SMBG) periodically. In order for such patients to be able to collect their own blood in a safe and secure manner, some blood collecting instruments have conventionally been used.

Such blood collecting instruments generally have a structure, as described in Patent Publications Nos. JP-B-2635823 and JP-B-3964457 (Patent Documents Nos. 1 and 2), wherein a lancet having a sharp piercing body such as a piercing needle or a blade and the like is stored together with a spring assembly in a housing with an opening that opens outward. Then, the lancet is biased or energized by the spring assembly so that the tip portion of the piercing body projects out through the housing's opening so as to perform a piercing operation. The configuration is such that this allows the piercing body to pierce through the skin surface pressed against by the housing's opening, enabling a small amount of blood to be exuded.

Meanwhile, disposable-type blood collecting instruments that are disposed after a single use have been widely used in recent years in order to prevent infections with HIV or B-type hepatitis viruses spread via used piercing bodies. Many of these disposable blood collecting instruments adopt a structure whereby the lancet is blocked from restoring to its standby position prior to the piercing operation (a position where the tip portion of the piercing body is stored in the housing under biased conditions of the lancet being pressed by the force of the spring assembly) once the action takes place.

For this reason, a user of such disposable blood collecting instrument sometimes had to undergo an operation of further exuding the blood, when the amount of exuded blood is checked by means of removing the housing away from the skin surface after the piercing operation and found insufficient for monitoring its blood glucose level, for example. In this situation, the user presses the surrounding area of the pierced skin again against the peripheral portion of the housing's opening. However, when the housing touches the blood on the skin surface in forms of spherical droplets due to surface tension, the blood droplets are often collapsed to spread over the skin, thus making it impossible to perform prescribed inspections and measurements of blood glucose levels and so forth.

### DOCUMENTS OF BACKGROUND ART

### PATENT DOCUMENTS

Patent Document 1: Patent Publication No. JP-B-2635823
Patent Document 2: Patent Publication No. JP-B-3964457

### SUMMARY OF THE INVENTION

### PROBLEM THE INVENTION ATTEMPTS TO SOLVE

The present invention has been created considering the circumstances described above as the background, and its object is to provide a disposable blood collecting instrument that is improved so as to be able to confirm whether the amount of blood exuded over the skin surface by piercing the skin is sufficient or not without removing the housing away from the skin surface, thereby securing enough amount of blood in a single piercing operation.

### MEANS FOR SOLVING THE PROBLEMS

Modes of the present invention designed to solve the above problem are described below. The structural elements adopted in each of the described modes are adoptable in as many combinations as possible.

One mode of the present invention provides a disposable blood collecting instrument including a lancet having a sharp piercing body at a tip thereof, and a spring assembly stored together with the lancet in a housing, a tip portion of the piercing body being arranged to protrude out of an opening of the housing to perform a piercing operation by means of a biasing force generated by the spring assembly, and the tip portion of the piercing body being arranged to retract to a recessed position within the housing after the piercing operation, the disposable blood collecting instrument being characterized in that: a surrounding area of the opening of the housing is made to be a visible portion with transparency that allows a view of an inside; and the spring assembly is located on a retracting side of the piercing body from the tip portion thereof when the lancet is retracted to the recessed position.

According to the present invention, since the surrounding area of the housing opening is made to be a visible portion, the pierced portion of the skin surface is visually identifiable through the visible portion when a piecing action takes place under the condition where the housing opening is pressed against the skin surface. Therefore, the amount of blood exuded from the pierced portion of the skin surface can be checked without removing the housing away from the skin surface. In addition, due to the position of the spring assembly stored in the housing, there is no difficulty in viewing the pierced portion of the skin surface through the visible portion, without being blocked by the spring assembly.

Therefore, by using the disposable blood collecting instrument according to the present invention, the amount of exuded blood can easily be checked without removing the housing away from the skin surface even if the blood is not sufficiently exuded therefrom by a single piercing operation. Also, when the amount of blood is not enough, more blood can be exuded from the pierced portion by means of keeping the housing at the same position as when the piercing operation was performed prior thereto without removing it away from the skin surface and forcibly pressing the surrounding area of the housing opening against the skin surface. At this moment, since the housing is kept at the same position, a situation can effectively be avoided where the housing touches the blood on the skin surface in forms of spherical droplets due to surface tension. Accordingly, the user can favorably increase the amount of blood to be collected while securely keeping the blood in droplet forms. This makes it possible to surely perform prescribed inspections and measurements of blood glucose levels and the like after a single piercing operation.

According to the present invention, another mode of the disposable blood collecting instrument can be adopted wherein a latching member is provided between the lancet and the housing in order to keep the lancet at a standby position against the biasing force of the spring assembly where the tip portion of the piercing body is stored in the housing under a condition where the lancet is biased by the spring assembly in a protruding direction of the piercing body; and a delatching member is provided in order to release the lancet held by the latching member from the standby position by an operation from outside the housing.

According to this mode, the user is able to perform the piercing action simply by actuating the delatching member without a need for any preparation work such as compressing the spring assembly and the like. This allows the user to collect his/her own blood more easily.

Also, in the event of adopting the mode in which the piercing action is performed by a combined mechanism of the latching and delatching members as described above, yet another mode can be adopted wherein a flexible arm having a free-end tip is provided to the lancet such that the free-end tip is deformable by deflecting in a direction of nearing an outer periphery of the lancet, and a latching portion is provided in the housing whereby the flexible arm is latched when the lancet is arranged in the standby position, whereby the latching member comprises the flexible arm and latching portion, and wherein the delatching member is configured such that the flexible arm is released from the latching portion by having the free-end tip of the flexible arm deflect toward the outer periphery of the lancet by an operation from outside.

According to this mode, it is now possible to form the flexible arm as a latching member that keeps the lancet at the standby position through an effective latching operation and an easy delatching operation. In addition, since the amount of protrusion of the flexible arm from the lancet toward the outer periphery can be minimized, it is now possible to make the outer size of the housing smaller and more compact.

Also, in the event that the latching member comprises the flexible arm and latching portion, another mode can favorably be adopted wherein the flexible arm is provided to extend along the lancet from a front side toward a rear side of the lancet in the protruding direction of the piercing body, the flexible arm is latched by the latching portion in a midpoint of the flexible arm in an extension direction thereof, and an operating portion that operates the delatching member from outside is provided on a tip side of the flexible arm in the extension direction thereof from a latched position where the flexible arm is latched by the latching portion.

This prevents the delatching member from touching the flexible arm when the lancet moves from the standby position toward its protruding direction once the latching condition at the latching portion of the flexible arm is released by the delatching member. Therefore, reduction in the thrusting force of the piercing body beyond the housing opening due to interference with the lancet by the delatching member can effectively be avoided when the lancet moves within the housing in the protruding direction of the piercing body beyond the housing opening. Moreover, since the operating portion for the delatching member is provided on the tip side of the flexible arm, it is now possible to release the latching of the flexible arm by deflecting it with a small operational force.

Also, according to the present invention, another mode can favorably be adopted wherein a cover portion that covers the tip portion of the piercing body is provided in a way detachable from the tip of the lancet, and an engaging portion is provided in the cover portion, keeping the lancet at the standby position against the biasing force of the spring assembly based on an action of engaging with the housing where the tip portion of the piercing body is arranged within the housing under the condition where the lancet is biased by the spring assembly in the protruding direction of the piercing body.

This prevents the tip portion of the piercing body from protruding beyond the housing's opening even if the engagement of the lancet by the latching member at the standby position is released due to an erroneous operation or a malfunction of the delatching member, unless the cover portion is detached from the tip portion of the lancet. Therefore, unexpected protrusion of the tip portion of the piercing body can surely be prevented by a simple and inexpensive structure that provides an engaging portion in the cover portion. As a result, safety in using or transporting the instrument can be secured in a very favorably way.

### EFFECT OF THE INVENTION

With the present invention, it is possible to check if the amount of blood exuded over the skin surface by a piercing operation is large enough without removing the housing away from the skin surface. This allows more blood to be exuded by means of pressing the surrounding area of the housing opening against the skin surface while keeping the surrounding area of the housing opening at the same position as when the piercing operation was performed prior thereto when the amount of blood was found insufficient. Then, a prescribed amount of blood can easily and securely be collected by a single piercing operation, which consequently makes it possible to surely and securely perform prescribed inspections and measurements of blood glucose levels and the like using the collected blood.

### BRIEF DESCRIPTION OF THE DRAWINGS

FIG.1 is a front view of a disposable blood collecting instrument as one embodiment of the present invention.
FIG. 2 is an exploded perspective view of the disposable blood collecting instrument shown in FIG. 1.
FIG. 3 is a cross sectional view taken along line 3-3 of FIG. 2.
FIG. 4 is a cross sectional view taken along line 4-4 of FIG. 1.
FIG. 5 is a cross sectional view taken along line 5-5 of FIG. 4.
FIG. 6 is a view that corresponds to FIG. 4 showing a pre-use standby condition of the disposable blood collecting instrument shown in FIG. 1.
FIG. 7 is a view that corresponds to FIG. 4 showing a use condition of the disposable blood collecting instrument shown in FIG. 1 after the piercing action is performed.
FIG. 8 is a cross sectional view taken along line 8-8 of FIG. 7.
FIG. 9 is a view that corresponds to FIG.4 showing an after-use condition of the disposable blood collecting instrument shown in FIG. 1 under the condition where a piercing needle is retracted to a recessed position.

### EMBODIMENTS FOR CARRYING OUT THE INVENTION

In order to further clarify the specifics of the present invention, embodiments thereof will be explained in detail below while referring to the drawings.

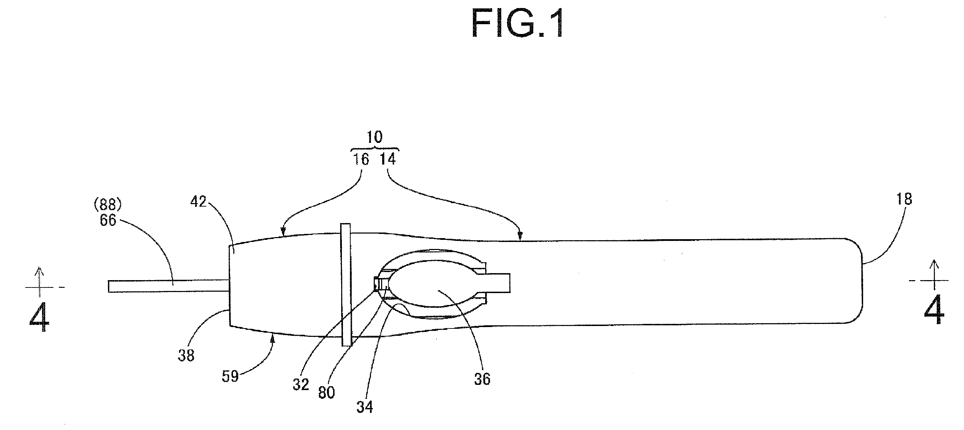

First, FIGS. 1 through 3 show a disposable blood collecting instrument as one embodiment of the present invention (hereinafter referred to simply as "blood collecting instrument"). The blood collecting instrument comprises a housing 10 made of synthetic resin, in which a lancet 11 and a compression coil spring 12 as a spring assembly are stored. In the descriptions below, unless indicated otherwise, the axial direction means a left-right direction in FIG.1, the front means the left side in FIG.1, and the rear means the right side in FIG.1.

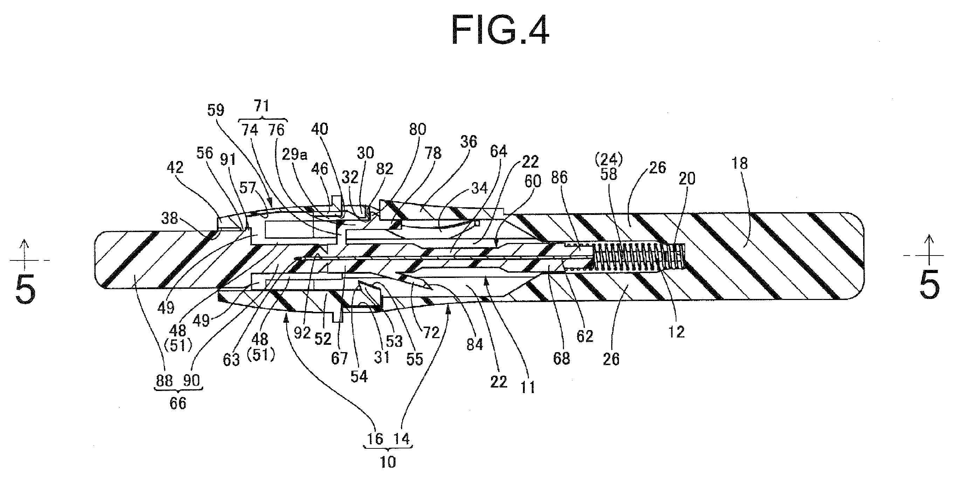

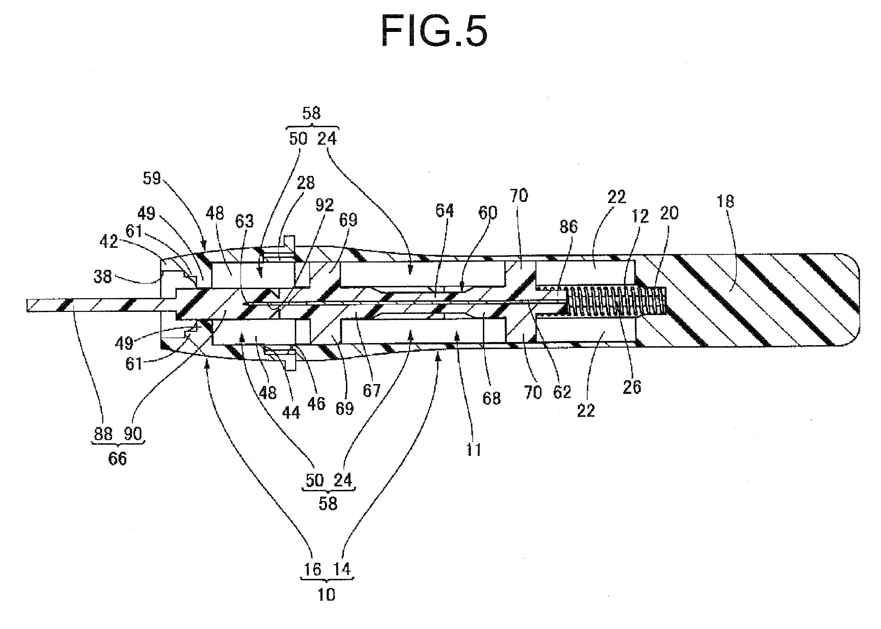

More specifically, as shown in FIGS.4 and 5, the housing 10 comprises a housing body 14 and a cylindrical cap 16 attached to the front end of the housing body 14. The housing body 14 is composed of a molding compound made from various resin materials including polyolefins such as polyethylene, polypropylene and ethylene-vinyl acetate copolymer, polyvinyl chloride, polyurethane, polystyrene, polymethylmethacrylate, polycarbonate, polyamide, polyesters such as polyethylene terephthalate and polybutylene terephthalate, acrylic resins, ABS resins, ionomer, polyacetal, polyphenylene sulfide, polyetheretherketone. Also, the housing body 14 has an overall form of an approximate cylinder closed on one side with an opening at the front end and a thick bottom portion 18 at the rear end. At the interior center of the bottom portion 18, a mounting hole 20 is provided in a shallow and circular form.

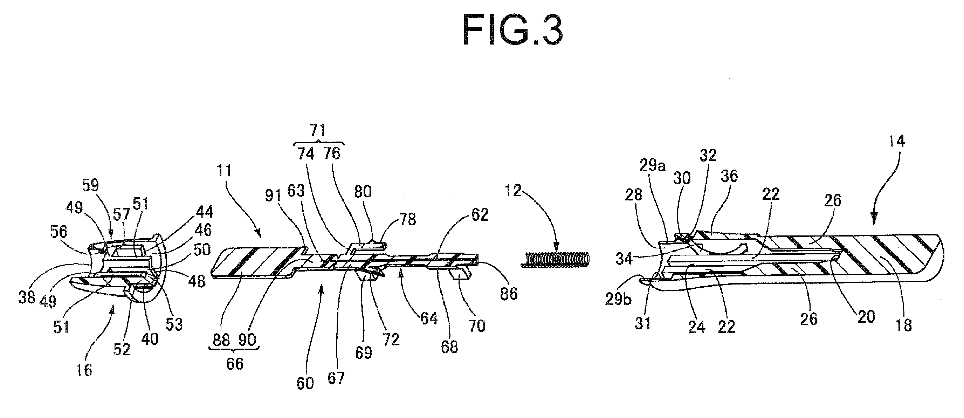

As evident from FIGS.3 through 5, two pairs of groove-forming ribs 22 totaling four are integrally provided protruding at two locations facing each other in the axis-perpendicular (radial) direction on the inner periphery of the housing body 14. These groove-forming ribs 22, facing each other at a given distance in a pair, in the peripheral direction of the housing body 14, consecutively protrude out in the axial direction. Also, each of these groove-forming ribs 22 has a length stretching from the bottom portion 18 of the housing body 14 to the proximity of the opening thereof. This allows each of groove portions 24 with its two sides corresponding to the opposing surfaces of the pair of groove-forming ribs 22 and 22 to be formed in such a way that it protrudes out consecutively in the axial direction for almost the entire length of the housing body 14.

On the inner periphery of the housing body 14, guide ribs 26 in a form of flat plates are each provided integrally to protrude out in the axial direction at two locations of the two groove portions 24 with a phase difference of 90 degrees with each other in the peripheral direction. These two guide ribs 26 and 26 have a length stretching from the bottom portion 18 of the housing body 14 all the way to the midpoint along the axis. Also, the tip surfaces of the protrusions thereof are arranged so as to oppose each other at a given distance.

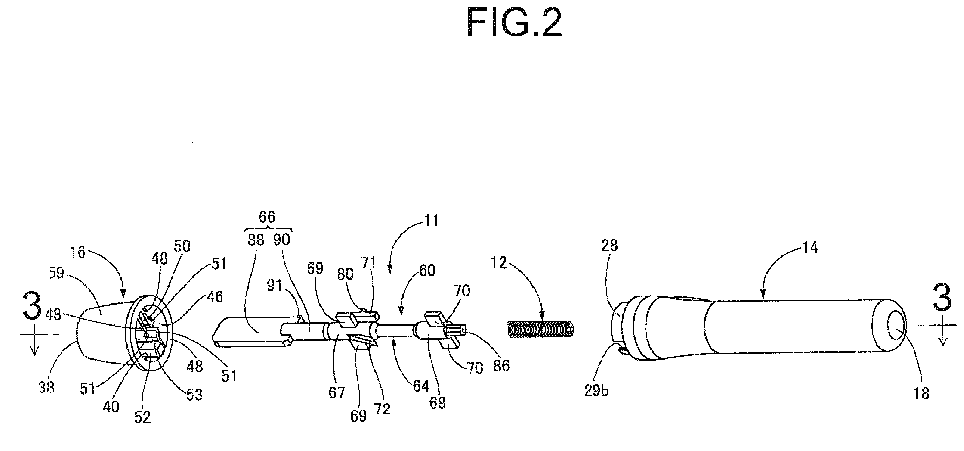

As shown in FIGS. 2 and 3, a cylindrical joint 28 in a form of a thin cylinder is integrally provided protruding on the same axis at the front end of the housing body 14. In this cylindrical joint 28 at the locations on the front side of the above two guide ribs 26 and 26 in the axial direction, notches 29a and 29b that extend across the whole length of the cylindrical joint 28 are each provided.

As shown in FIGS.3 and 4, on the inner periphery of the front end of the housing body 14 located on the back side of the notch 29a in the axial direction, a latching protrusion 30 is provided as a latching portion. This latching protrusion 30 is in an approximate form of a right triangle in longitudinal cross section and its rear lateral side is made to be a latching surface 32 that extends in the axis-perpendicular direction. In addition, on the cylindrical wall of the housing body 14 located on the rear side of the latching protrusion 30 in the axial direction, a slot 34 in an approximate form of reverse U-shape is provided that extends in the axial direction (see FIG.1). Then, the cylindrical portion surrounded by the slot 34 is made to form an operating portion 36 that is elastically deformable in the axis-perpendicular direction due to the use of a synthetic resin spring. This operating portion 36 gets thicker outward in the axis-perpendicular direction in order to easily distinguish itself from other portions of the housing body 14. Also, on the inner periphery of the front end of the housing body 14 located toward the back of the notch 29b in the axial direction, a narrow joint groove 31 is formed to extend comparatively a short distance in the axial direction.

On the other hand, as evident from FIGS.2 through 5, the cylindrical cap 16 attached to the front end of the housing body 14 is formed, as a whole, in an approximate cylinder having a substantially shorter length than the housing body 14 in the axial direction and the same inner radius as that of the housing body 14, and its openings on both sides in the axial direction are made to form a front opening 38 and a rear opening 40 respectively. The cylindrical cap 16 is formed of a tapered shape wherein the outer periphery on the front end side opposite from the mounting side of the housing body 14 gets smaller in radius toward the front. This makes the front end of the cylindrical cap 16 and hence the front end of the housing 10 to form a thin end portion 42 which is made thinner than other portions.

As shown in FIGS. 3 and 5, a step portion 44 is formed that increases the inner radius of the rear end side of the cylindrical cap 16 on the inner periphery thereof around the midpoint in the axial direction. Then, further back from the step portion 44 with increased inner radius is made to form a joint portion 46.

As shown in FIGS. 2, 3 and 5, two pairs of groove-forming ribs 48 and 48 totaling four are integrally provided at two locations facing each other in the axis-perpendicular (radial) direction on the inner periphery of the cylindrical cap 16. These groove-forming ribs 48 are formed in similar shapes to those of the two pairs of groove-forming ribs 22, 22, 22 and 22 provided on the inner periphery of the housing body 14. This makes a groove portion 50 to form, at each of two locations facing each other in the axis-perpendicular direction on the inner periphery of the cylindrical cap 16, with its both sides corresponding to the opposing surfaces of the pair of groove-forming ribs 48 and 48 in a way of consecutively extending in the axial direction with the same width as that of the groove portion 24 of the housing body 14.

Meanwhile, these groove-forming ribs 48 each have a length stretching from a position offset backward in a given dimension from the end surface of the front opening 38 of the cylindrical cap 16 all the way to the end surface of the rear opening 40. At each front end of the groove-forming ribs 48, a stopper rib 49 is integrally provided. Each of these stopper ribs 49 extends out from the opposite side of the opposing surface of the pair of groove-forming ribs 48 in the axis-perpendicular direction, interconnecting each groove-forming rib 48 and the inner periphery of the cylindrical cap 16. In addition, at the tip portion of the protrusion of each groove-forming rib 48, each cylinder division 51 is integrally provided.

As evident from FIGS.2 through 4, in the rear end of the cylindrical cap 16 at one location having a phase difference of 90 degrees from either of the two groove portions 50 in the peripheral direction, a stopper-tab-forming protrusion 52 is integrally provided so as to protrude a given distance from the rear opening 40 in the axial direction. At the tip portion of this stopper-tab-forming protrusion 52, a stopper tab 53 is integrally formed to protrude inward in the radial direction of the cylindrical cap 16. The stopper tab 53 is in an approximate form of a right triangle in longitudinal cross section, and its front lateral side in the axial direction is made to form a stopper surface 54 that extends out in the axis-perpendicular direction of the cylindrical cap 16, whereas its rear lateral side in the axial direction are made to form a sliding surface 55 that extends out slantedly so as to gradually lower the protrusion height of the stopper tab 53 toward the tip side of the stopper-tab-forming protrusion 52.

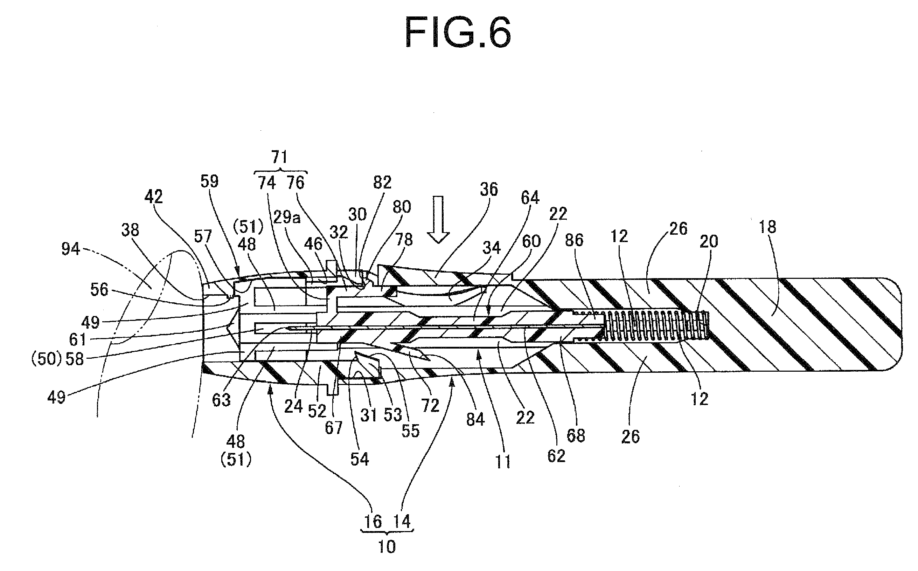

Meanwhile, as evident from FIG. 4, on the inner periphery of the cylindrical cap 16 at the front end of the portion radially opposite the forming portion of the stopper-tab-forming protrusion 52, an engaging convex portion 56 is integrally formed. This engaging convex portion 56 comprises a quadrangular shape protrusion that protrudes out in the axis-perpendicular direction at a rather low height from the inner periphery of the cylindrical cap 16. Also, two sides facing each other in the front-back direction among the four sides are arranged so as to extend in the axis-perpendicular direction. In addition, on the inner periphery of the engaging convex portion 56 of the cylindrical cap 16 toward the back in the axial direction, a holding groove 57 is provided to extend in the axial direction. This holding groove 57 has a bottom surface continuous from the inner periphery of the above joint portion 46 without any level difference. In addition, as evident from FIGS.5 and 6, on the inner periphery of the cylindrical cap 16 toward the front end, a pair of guiding convex portions 61 are formed facing each other at locations having a phase difference of 90 degrees from each engaging convex portion 56 in the peripheral direction. The guiding convex portions 61 protrude out in the axis-perpendicular direction at a rather low height from the inner periphery of the cylindrical cap 16, as does the engaging convex portion 56, to form a triangle with an apex toward the front in the axial direction on the inner periphery of the cylindrical cap 16.

Then, as shown in FIGS.4 and 5, the joint portion 46 of the cylindrical cap 16 having the above-mentioned structure is made to fit onto the cylindrical joint 28 at the front end of the housing body 14 and fixed thereto. This allows the housing 10 to be composed as an integral assembly of the cylindrical cap 16 and housing body 14. Thus, the housing 10 opens forward via the front opening 38 of the cylindrical cap 16, while being formed in an approximate shape of a cylinder closed on one side at the rear end by the bottom portion 18 of the housing body 14.

Meanwhile, under the fixed condition of the cylindrical cap 16 and the housing body 14, two groove portions 50 and 50 provided on the inner periphery of the cylindrical cap 16 are arranged so as to continuously align with the two groove portions 24 and 24 formed along the periphery of the housing body 14. This allows guiding grooves 58 comprising groove portion 24 and 50 of the housing body 14 and the cylindrical cap 16, respectively, to be formed at two locations facing each other in the axis-perpendicular direction on the inner periphery of the housing 10 so as to continuously extend in the axial direction at a length stretching from the bottom portion 18 to the proximity of the front opening 38.

Also, under the fixed condition of the cylindrical cap 16 and the housing body 14, the engaging convex portion 56 and the holding groove 57 provided to align in front and back in the axial direction on the inner periphery of the cylindrical cap 16 are arranged on the front side of the notch 29a formed at the latching protrusion 30 and the cylindrical joint 28 provided at the front end of the housing body 14. In addition, the stopper-tab-forming protrusion 52 that protrudes from the rear opening 40 of the cylindrical cap 16 is inserted into the notch 29b formed at the cylindrical joint 28 of the housing body 14, while being inserted into the joint groove 31 provided on the rear side of the notch 29b in the axial direction. Thus, the stopper tab 53 provided to project out at the stopper-tab-forming protrusion 52 is protruded from the inner periphery at the front end of the housing body 14. This allows the stopper surface 54 of the stopper tab 53 and the sliding surface 55 of the stopper tab 53 to be arranged on the front side and rear side, respectively, of the inner periphery at the front end of the housing body 14. This stopper surface 54 of the stopper tab 53 is tapered at a steep angle toward the rear from the tip to base side of the stopper tab 53. The sliding surface 55 is tapered at a moderate angle toward the rear from the tip to base side of the stopper tab 53.

Then, in this embodiment, especially the cylindrical cap 16 fixed to the front end of the housing body 14 is composed of a transparent molding compound made from various resin materials including polyethylene, polypropylene, polyvinyl chloride, polyurethane, polystyrene, polymethylmethacrylate, polycarbonate, polyethylene terephthalate, acrylic resins, ABS resins and so forth. This allows the front part of the housing 10 composed of the cylindrical cap 16, that is the surrounding area of the front opening 38 of the housing 10, or more specifically, the front side of the joint portion 46 of the cylindrical cap 16 that gets fit onto the cylindrical joint 28 of the housing body 14, is made to form a visible portion 59 that allow a view of the inside from the outside. Here, the transparency is deemed enough if the inside is visible from the outside, and being clear and tinted with color is permissible as well as being clear and colorless as long as the visibility is established as described above.

Meanwhile, as shown in FIGS.2 through 5, the lancet 11 includes a lancet hub 60 and a piercing needle 62 as a sharp piercing body. The lancet hub 60 is made from resin compounds such as polypropylene, polyethylene, polycarbonate, ABS resins and acrylic resins and the like. Also, the lancet hub 60 includes a needle holder 64 in an approximate form of a long and thin rod that is insertable into the housing 10 and a cover cap 66, as a cover portion, integrally formed at the front end of the needle holder 64 in a way easily detachable therefrom. Then, the piercing needle 62 is coaxially fixed to the needle holder 64 by insert molding or adhesion under a condition where a needlepoint 63, as a tip portion of the piercing body, protrudes out from the front end of the needle holder 64 on the central axis. Also, the needlepoint 63 of the piercing needle 62 that protrudes out from the needle holder 64 is entirely covered by the cover cap 66.

At the front (tip side) and rear (base side) portions of the needle holder 64 of the lancet hub 60, a front larger-diameter portion 67 and a rear larger-diameter portion 68 are respectively formed. Two sliding ribs 69 and 69 are integrally provided with the front larger-diameter portion 67 to protrude therefrom, whereas sliding ribs 70 and 70 are integrally provided with the rear larger-diameter portion 68 to protrude therefrom. These sliding ribs 69 and 70 are each in a form of a flat plate that can fit, in a slidable way, in the above guiding grooves 58 and 58 provided on the inner periphery of the housing 10, and protrude out in the axis-perpendicular direction from two locations that have a phase difference of 180 degrees in the peripheral direction along the outer periphery of each of the front larger-diameter portion 67 and the rear larger-diameter portion 68.

Also, as shown in FIGS. 2 through 4, a first flexible arm 71 and a second flexible arm 72 are provided integrally with the front larger-diameter portion 67 of the needle holder 64 to protrude therefrom. The first flexible arm 71, generally in a form of a hooked plate, integrally comprises a protruded plate 74 that protrudes out integrally from the outer periphery of the front larger-diameter portion 67 at a given height in the axis-perpendicular direction of the needle holder 64 and an extended plate 76 that is located laterally apart from the needle holder 64 by a prescribed distance and extends out parallel to the needle holder 64 from the tip of the protruded plate 74 toward the rear end of the needle holder 64. Such a tip of the extended plate 76 is made to be a free end. This allows the first flexible arm 71 to flexurally (elastically) deform based on the use of a synthetic resin spring in the direction where the tip of the extended plate 76, being made to be a free end, gets closer to or farther from the outer periphery of the needle holder 64. Here, the first flexible arm 71 is arranged with a phase difference of 90 degrees from each of the two sliding ribs 69 and 69 in the peripheral direction along the periphery of the front larger-diameter portion 67.

Also, as to the first flexible arm 71 described above, the tip of the extended plate 76 being a free end is made to be an engagement end 78, whereas an engaging tab 80 is integrally provided protruding at the location on the base end side (closer to the protruded plate 74) of this engagement end 78. The engaging tab 80 is in a cross-sectional form of a right triangle that is provided on the inner periphery of the housing body 14. Among all sides of the engaging tab 80, the one located on the base end side of the first flexible arm 71 is made to be an engagement surface 82 that extends in the axis-perpendicular direction of the needle holder 64 corresponding to the latching surface 32 of the latching protrusion 30.

The second flexible arm 72 protrudes out from the outer periphery of the front larger-diameter portion 67 so as to extend out slantedly while getting away gradually from the front larger-diameter portion 67 toward the rear end of the needle holder 64. This second flexible arm 72 has its protruded tip portion as a free end. This allows the second flexible arm 72, as the first flexible arm 71, to flexurally (elastically) deform based on the use of a synthetic resin spring in the direction where the tip portion, being made to be a free end, gets closer to or farther from the outer periphery of the needle holder 64. Then, the tip end surface of the second flexible arm 72 is made to be an engagement surface 84 that extends slantedly to the front side of the needle holder 64 toward the outer periphery thereof corresponding to the stopper surface 54 of the stopper tab 53 provided at the stopper-tab-forming protrusion 52 of the cylindrical cap 16. Here, the second flexible arm 72 is arranged with a phase difference of 180 degrees from the first flexible arm 71 in the peripheral direction of the front larger-diameter portion 67.

On the rear surface of the rear larger-diameter portion 68 of the needle holder 64, a spring fixing portion 86 is integrally provided protruding therefrom. This spring fixing portion 86 has a form in which protruding ridges that extends in the axial direction are each integrally formed at four locations with equal intervals in the peripheral direction along the outer periphery of a cylinder body that is short in the axial direction.

Meanwhile, as shown in FIGS.2 through 5, the cover cap 66 integrally formed at the front end of the needle holder 64 comprises a grip portion 88 in a form of a longitudinal flat plate and a cover part 90 in an approximate form of a round rod in an integral manner. As evident from FIG.4, at the rear end portion of one side of the grip portion 88 in its width direction, an engaging portion 91 is integrally provided protruding therefrom. This engaging portion 91 is composed of a quadrangle-shape protrusion corresponding to the engaging convex portion 56 provided at the cylindrical cap 16 of the housing 10. Then, two opposing sides in the front-back direction among the four sides of the engaging portion 91 are arranged so as to extend in the axis-perpendicular direction.

The cover part 90 integrally extends out from the center in the width direction of one end of the grip portion 88 in its longitudinal direction. In this cover part 90, a small-diameter pore 92 that opens up on the end surface opposite the grip portion 88 is formed.

Then, the cover cap 66 is integrally connected to the front end of the needle holder 64 at the other end of the grip portion 88 of the cover part 90. Under the connected condition of these cover cap 66 and needle holder 64, the needlepoint 63 of the piercing needle 62 that protrudes out from the front end of the needle holder 64 is stored in the small-diameter pore 92 of the cover part 90, which covers the entire needlepoint 63. Also, at the joint portion between the cover cap 66 and the needle holder 64, a V-shape groove notch is provided all the way around. This allows the cover cap 66 including the cover part 90 to be easily detached from the front end of the needle holder 64, for example by holding the grip portion 88 and twisting it by hand. Meanwhile, in such a detaching operation, the engaging portion 91 provided at the grip portion 88 can be disengaged from the engaging convex portion 56 provided at the cylindrical cap 16 of the housing 10 by means of twisting the grip portion 88 so as to turn it in the circumferential direction. At this moment, as the grip portion 88 turns in the circumferential direction, the engaging portion 91 is made to abut against the guiding convex portion 61 provided protruding on the inner periphery of the cylindrical cap 16. As shown in FIG.6, this guiding convex portion 61 is formed in a triangle with an apex toward the front in the axial direction, and as the grip portion 88 turns, the engaging portion 91 is guided toward the front along the slope of the guiding convex portion 61 toward the front in the axial direction to move over to the front side of the engaging cover portion 56 in the axial direction. This prevents the engaging portion 91 of the grip portion 88 and the engaging convex portion 56 of the cylindrical cap 16 from being engaged with each other again, even if the grip portion 88 is turned 360 degrees in the circumferential direction, ensuring that the engaging portion 91 and the engaging convex portion 56 are disengaged by the turning operation of the grip portion 88. Also, the detachment of the cover cap 66 from the needle holder 64 allows the needlepoint 63 of the piercing needle 62 to protrude out from the front end of the needle holder 64 to be exposed outside.

In addition, as evident from FIGS.4 and 5, the lancet 11 having the above structure is coaxially inserted into the housing 10 to be stored therein. Also, under such a condition of storing, the needle holder 64 is arranged within the housing body 14, while the cover cap 66 is arranged within the cylindrical cap 16 under a condition where the grip portion 88 is protruded forward from the front opening 38 of the cylindrical cap 16. Then, the four sliding ribs 69, 69, 70 and 70 of the needle holder 64 are each fit in the guiding grooves 58 and 58 provided on the inner periphery of the housing 10 in a slidable way. This allows the lancet 11 in the housing 10 to slide in the axial direction thereof while being guided by each guiding groove 58 without being able to turn around the axial center.

The cover cap 66 placed in the cylindrical cap 16 is arranged in such a way that the cover part 90 is surrounded from outside by each cylinder division 51 of the four groove-forming ribs 48, 48, 48 and 48 provided on the inner periphery of the cylindrical cap 16.

Also, under such a condition where the lancet 11 is placed in the housing 10, the compression coil spring 12 is arranged so as to fit between the opposing surfaces of the two guide ribs 26 and 26 between the needle holder 64 of the lancet 11 located within the housing body 14 and the bottom portion 18 of the housing body 14. This compression coil spring 12 is fixed under a condition where one of its ends in the axial direction is fit onto the spring fixing portion 86 of the needle holder 64. Meanwhile, the other end in the axial direction is pressed into the above mounting hole 20 provided at the interior center of the bottom portion 18 of the housing body 14 to be fixed thereto.

This allows the lancet 11 stored in the housing 10 to be mounted to the bottom portion 18 of the housing body 14 via the compression coil spring 12 so as to compose the blood collecting instrument of the present embodiment. Then, in this blood collecting instrument, the lancet 11 is made slidable in the front-back direction along the axial direction in the housing 10. Also, when the lancet 11 moves forward as the compression coil spring 12 expands after the cover cap 66 is detached from the needle holder 64, the needlepoint 63 of the piercing needle 62 fixed to the lancet 11 (needle holder 64) is made to protrude forward from the front opening 38 of the housing 10 (cylindrical cap 16).

The blood collecting instrument of the present embodiment with the above structure is provided to a user in an unused initial state under a condition where the needlepoint 63 of the piercing needle 62 is covered by the cover part 90 of the cover cap 66 while the entire lancet 11 is stored in the housing 10.

In other words, as shown in FIGS.4 and 5, in the unused initial state, the needlepoint 63 of the piercing needle 62 covered by the cover part 90 of the cover cap 66 is arranged so as to be positioned within the rear end of the cylindrical cap 16 of the housing 10. At this moment, the spring fixing portion 86 of the needle holder 64 is placed closer to the interior surface of the bottom portion 18 of the housing body 14. This makes the compression coil spring 12 to be compressed to bring the lancet 11 in a biased condition toward the front due to the compression coil spring 12.

Also, under such a condition, the engagement end 78 of the first flexible arm 71 is in contact with the front end of the interior surface in the operating portion 36 of the housing body 14. In addition, the engaging tab 80 of the first flexible arm 71 thrusts itself into the slot 34 provided in the housing body 14. Also, the engagement surface 82 of the engaging tab 80 is arranged on the rear side of the latching surface 32 of the latching protrusion 30 in the housing body 14 with a slight gap in between. Then, the front surface of the engaging portion 91 of the cover cap 66 is engaged with the rear surface of the engaging convex portion 56 provided protruding on the inner periphery on the front end side of the cylindrical cap 16.

In the initial state of the blood collecting instrument of the present embodiment, this allows the lancet 11 to be biased in the direction of being moved forward by the compression coil spring 12, whereas the movement of the lancet 11 toward the front is blocked by the engagement of the engaging portion 91 of the cover cap 66 with the engaging convex portion 56 of the cylindrical cap 16. In other words, due to such an engagement between the engaging portion 91 of the cover cap 66 and the engaging convex portion 56 of the cylindrical cap 16, the lancet 11 is arranged in a standby position, which is the initial state, while the needlepoint 63 is prevented from protruding out from the opening of the housing 10.

Now, when a user such as a patient collects his/her own blood using the blood collecting instrument of the present embodiment in the initial state as described above, he/she can follow the following steps, for example:

First, twist off the cover cap 66 to be detached from the lancet hub 60 by means of holding and twisting the grip portion 88 that protrudes out from the front opening 38 of the housing 10 under the unused initial state as shown in FIGS.4 and 5. This allows the needlepoint 63 of the piercing needle 62 to be exposed outside within the cylindrical cap 16 of the housing 10 as shown in FIG. 6. At this moment, the engagement between the engaging portion 91 of the cover cap 66 and the engaging convex portion 56 of the housing 10 (cylindrical cap 16) is released. Then, the engaging tab 80 provided on the first flexible arm 71 of the lancet 11 and the latching protrusion 30 provided in the housing body 14 of the housing 10 are engaged with each other respectively at the engagement surface 82 and the latching surface 32, which allows the lancet 11 to be latched against the latching protrusion 30 of the housing 10 to prevent itself from moving forward.

Thus, under the condition of being prevented from moving forward by detaching the cover cap 66 from the initial state, the lancet 11 is placed at a position where the needlepoint 63 of the piercing needle 62 is stored in the front end of the housing 10 (cylindrical cap 16), that is a standby position. As evident from this, in the present embodiment, a latching member is composed of the latching protrusion 30 provided on the inner periphery of the housing 10 and the engaging tab 80 provided on the first flexible arm 71 of the lancet 11 so that the lancet 11 is latched against the housing 10.

Next, under a condition where the cushion of the finger 94 and the like is pressed against the front end surface of the thin end portion 42 of the housing 10, as shown by the outlined arrow in FIG.6, press the operating portion 36 provided in the housing 10 inward in the axis-perpendicular direction of the housing 10 and push the inside corner of the front edge of the operating portion 36 into the housing 10. This allows the engagement end 78 of the first flexible arm 71 to be pressed inward of the housing 10 by means of the operating portion 36 to flexurally deform the extended plate 76 of the first flexible arm 71, thus detaching the engaging tab 80 provided on the extended plate 76 from within the slot 34 to the inside the housing 10.

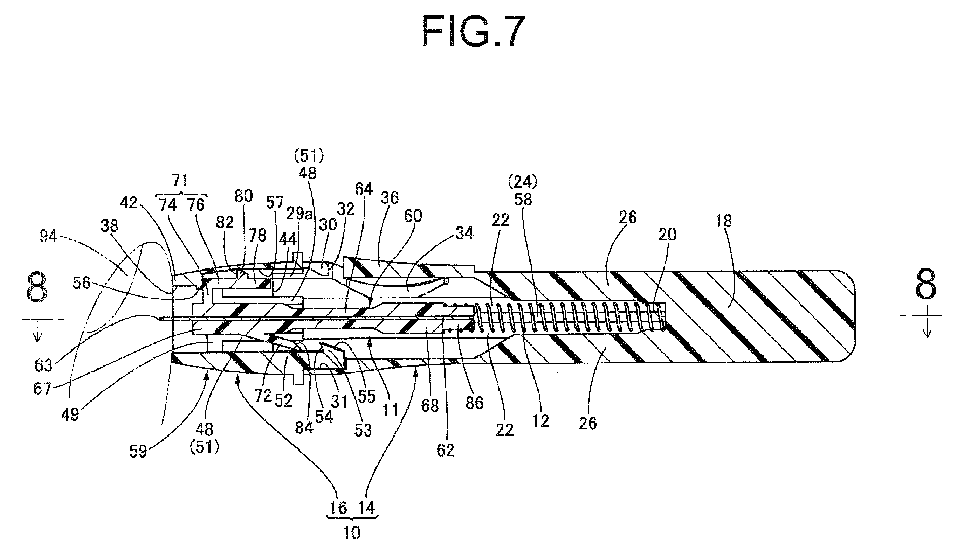

Then, as shown in FIG.7, release the latching condition between the engaging tab 80 and the latching protrusion 30, and move the lancet 11 forward based on the biasing force of the compression coil spring 12 to let the needlepoint 63 of the piercing needle 62 to protrude out from the front opening 38 of the housing 10. This allows the user to perform the piercing operation to pierce the needlepoint 63 of the piercing needle 62 into the finger 94 in order to exude out blood from the pierced portion thereof. As evident from the above, in the present embodiment, a delatching member is composed of the operating portion 36.

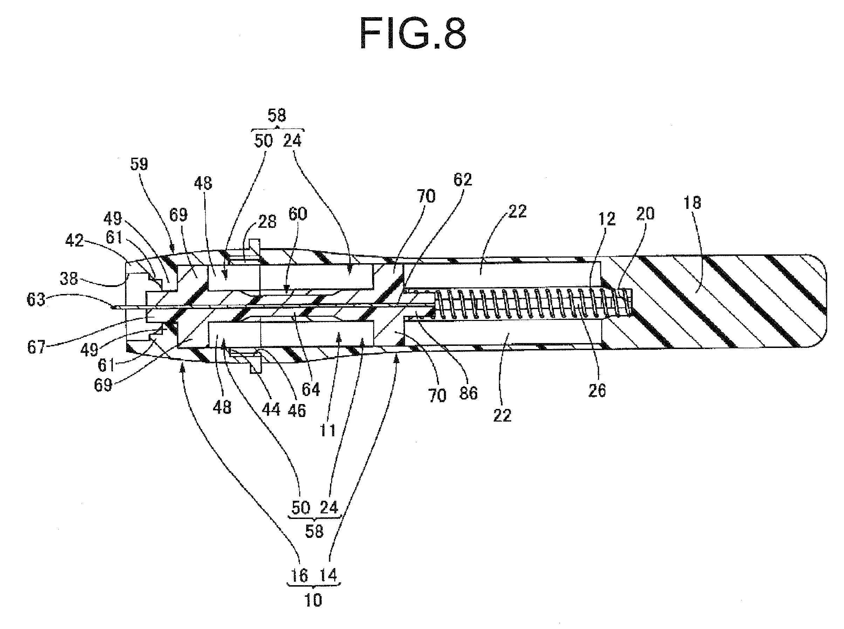

At this moment, the needlepoint 63 of the piercing needle 62 is made to protrude out instantaneously or temporarily from the front opening 38 of the housing 10 by means of having the compression coil spring 12 expand beyond its free length. Also, during the movement of the lancet 11, the tip end of the second flexible arm 72 goes over the stopper tab 53 while sliding over the sliding surface 55 of the stopper tab 53 that protrudes out on the inner periphery of the housing 10 so as to be placed on the front side of the stopper tab 53 when the needlepoint 63 of the piercing needle 62 protrudes out from the front opening 38. Then, as shown in FIG.8, the two sliding ribs 69 provided at the front larger-diameter portion 67 of the lancet 11 are abutted against the stopper ribs 49 and 49 provided at the front end of the cylindrical cap 16 to regulate the amount of protrusion of the needlepoint 63 of the piercing needle 62 from its front opening 38. This allows the user to perform the piercing operation in a safe way by keeping the amount of piercing constant. Also at this moment, the extended plate 76 of the first flexible arm 71 gets stored in the holding groove 57 of the cylindrical cap 16.

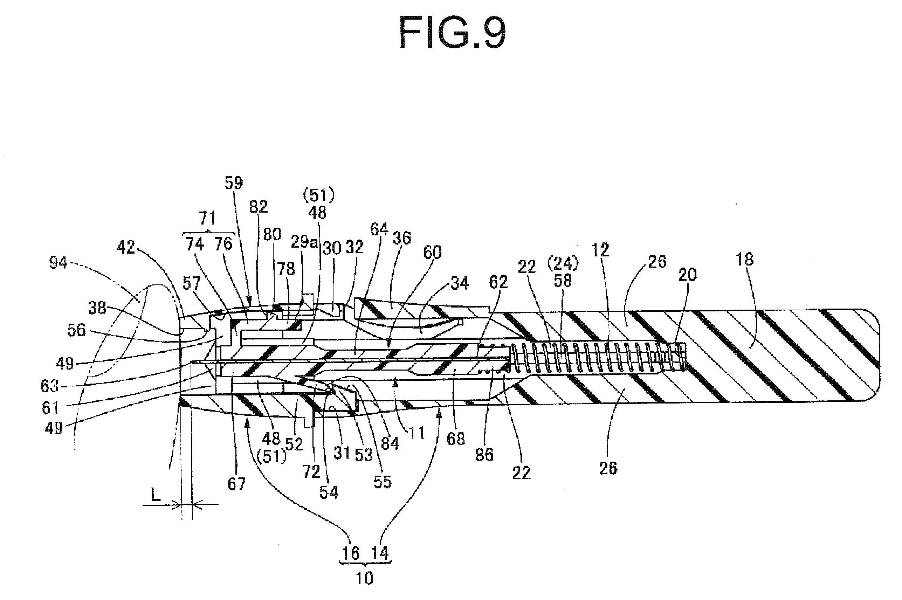

Then, after such a piercing operation by the instantaneous expansion of the compression coil spring 12, it gets contracted from the time of piercing operation by restoring to its natural state with no need for maneuvering as shown in FIG.9. This allows the lancet 11 to be placed at a recessed position where the needlepoint 63 of the piercing needle 62 is recessed within the housing 10 and stored within the visible portion 59 of the housing 10 comprising the front end of the cylindrical cap 16. At this moment, the engagement surface 84 of the second flexible arm 72 of the lancet 11 gets latched at the stopper surface 54 of the stopper tab 53 that protrudes out on the inner periphery of the housing 10. This prevents the lancet 11 from moving backward from the recessed position. As a result, it is made impossible to perform the piercing operation again and the reuse of the blood collecting instrument is disabled. Therefore, even if the operating portion 36 is pressed again after the piercing operation, there is no way that the needlepoint 63 of the piercing needle 62 protrudes out from the front opening 38 of the housing 10.

In the blood collecting instrument of the present embodiment, the distance L from the front end of the housing 10 to the needlepoint 63 when the lancet 11 is placed at the recessed position is made larger than that of conventional instruments. More specifically, such distance L is set at approximately 3 to 5mm.

Thus, by the blood collecting instrument of the present embodiment, one can perform a piercing operation, after twisting off the cover cap 66, simply by pressing the operating portion 36 under a condition where the finger 94 is in contact with the front end surface of the housing 10. Then, once the piercing operation is completed, the instrument is rendered non-reusable without having the needlepoint 63 of the piercing needle 62 protrude out from the front opening 38 of the housing 10.

Also, under an unused condition before the piercing operation, the needlepoint 63 of the piercing needle 62 is covered by the cover cap 66. In addition, unless the cover cap 66 is removed, the lancet 11 does not move forward even if the operating portion 36 is pressed for operation and the needlepoint 63 of the piercing needle 62 does not protrude out. This effectively enhances the user safety at a low cost of structure.

Furthermore, in the blood collecting instrument of the present embodiment, the front end of the housing 10 is made to be the visible portion 59 that can be seen through inside. For this reason, when performing the piercing operation, the user is able to check the amount of blood to be exuded from the pierced portion of the finger 94 from outside the visible portion 59. Additionally, since the housing body 14 or the compression coil spring 12 does not exist inside the visible portion 59, there is no way that they would interfere with the observation of the pierced portion of the finger 94.

Therefore, using such a blood collecting instrument, whether the amount of blood exuded from the finger 94 by a single piercing operation is sufficient or not can easily be confirmed via the visible portion 59. In doing that, the user can press the front end of the housing 10 hard against the finger 94 at the same position as the piercing operation without moving the front end of the housing 10 away from the finger 94 in order to exude more blood from the pierced portion. At this moment, since the position of the housing 10 is unchanged, one can effectively avoid such an incident wherein the housing 10 touches the blood on the skin surface in forms of spherical droplets due to surface tension.

Therefore, using the blood collecting instrument of the present embodiment, one can collect a sufficient amount of blood from the finger 94 by a single piercing operation while keeping the blood droplets in a stable condition. As a result, it is now possible to surely perform prescribed inspections and measurements such as checking blood glucose levels and the like after a single piercing operation.

Also, the blood collecting instrument of the present embodiment works in such a way that the engagement between the engaging tab 80 of the first flexible arm 71 and the latching protrusion 30 of the housing 10 is released to move the lancet 11 forward by means of having the front end portion of the operating portion 36 press the engagement end 78 which is in the rear end of the first flexible arm 71 provided protruding to extend toward the rear side from the front side. Therefore, when the lancet 11 moves forward, the reduction in the force of protrusion of the piercing needle 62 from the front opening 38 of the housing 10 due to the interference with the lancet 11 by the operating portion 36 can be effectively avoided. Accordingly, the piercing operation can be performed even more securely.

While one embodiment of the present invention have been described in detail above, the present invention is not restricted to those specific descriptions.

The blood collecting instrument of the present embodiment has a single compression coil spring 12 that both extends and retracts the piercing needle 62 stored in the housing 10, thereby achieving the simplification and cost saving of the structure in a favorable way, but it is also possible to store in the housing 10 both a spring assembly for protruding the piercing needle 62 and another for retracting the same, or a spring assembly for both protruding and retracting the piercing needle 62 together with another spring assembly for protrusion to complement the force of protrusion of the piercing needle 62. Of course, in that case, a configuration can be adopted wherein those two spring assemblies are stored within the housing 10 on the opposite rear side from the position of the needlepoint 63 of the piercing needle 62 in its protruding direction when the lancet 11 is placed at the recessed position.

Moreover, in lieu of the piercing needle 62, a blade and the like can be used as a piercing body.

Also, in lieu of the compression coil spring 12, a tension coil spring and various other kinds of springs can be used as a spring assembly.

In addition, the structures of the latching and detaching member are, of course, not restricted at all to those exemplified above.

The correlation between the two standby positions of the lancet, that are the "lancet's standby position" expressed by latching member provided between the lancet and housing that maintains the lancet at the standby position against the biasing force of the spring assembly and the "lancet's standby position" expressed by the engaging portion provided at the cover portion that maintains the lancet at the standby position against the biasing force of the spring assembly based on the engagement action with the housing is not restricted to any specific one in the present invention. For instance, the standby position expressed by the former latching member and the standby position expressed by the latter engaging portion can be set at the same place, or either one can be set in front of the other in the protruding direction of the piercing body. In either case, the fail-safe function provided by the latter engaging portion is exerted, and the lancet can be biased to displace itself in the protruding direction to perform the piercing operation by means of releasing the former latching member after releasing the holding mechanism of said engaging portion that keeps the lancet at the standby position.

### KEYS TO SYMBOLS

10: Housing, 11: Lancet, 12: Compression coil spring, 14: housing body, 16: Cylindrical cap, 30: Latching protrusion, 36: Operating portion, 38: Front opening, 56: Engaging convex portion, 59: Visible portion, 60: Lancet hub, 62: Piercing needle, 63: Needlepoint, 66: Cover cap, 71: First flexile arm, 80: Engaging tab, 91: Engaging portion

## Claims

1. A disposable blood collecting instrument including a lancet having a sharp piercing body at a tip thereof, and a spring assembly stored together with the lancet in a housing, a tip portion of the piercing body being arranged to protrude out of an opening of the housing to perform a piercing operation by means of a biasing force generated by the spring assembly, and the tip portion of the piercing body being arranged to retract to a recessed position within the housing after the piercing operation, the disposable blood collecting instrument being **characterized in that**:
a surrounding area of the opening of the housing is made to be a visible portion with transparency that allows a view of an inside; and
the spring assembly is located on a retracting side of the piercing body from the tip portion thereof when the lancet is retracted to the recessed position.

2. The disposable blood collecting instrument according to claim 1, wherein a latching member is provided between the lancet and the housing in order to keep the lancet at a standby position against the biasing force of the spring assembly where the tip portion of the piercing body is stored in the housing under a condition where the lancet is biased by the spring assembly in a protruding direction of the piercing body; and a delatching member is provided in order to release the lancet held by the latching member from the standby position by an operation from outside the housing.

3. The disposable blood collecting instrument according to claim 2, wherein a flexible arm having a free-end tip is provided to the lancet such that the free-end tip is deformable by deflecting in a direction of nearing an outer periphery of the lancet, and a latching portion is provided in the housing whereby the flexible arm is latched when the lancet is arranged in the standby position, whereby the latching member comprises the flexible arm and latching portion, and wherein the delatching member is configured such that the flexible arm is released from the latching portion by having the free-end tip of the flexible arm deflect toward the outer periphery of the lancet by an operation from outside.

4. The disposable blood collecting instrument according to claim 3, wherein the flexible arm is provided to extend along the lancet from a front side toward a rear side of the lancet in the protruding direction of the piercing body, the flexible arm is latched by the latching portion in a midpoint of the flexible arm in an extension direction thereof, and an operating portion that operates the delatching member from outside is provided on a tip side of the flexible arm in the extension direction thereof from a latched position where the flexible arm is latched by the latching portion.

5. The disposable blood collecting instrument according to any one of claims 1-4, wherein a cover portion that covers the tip portion of the piercing body is provided in a way detachable from the tip of the lancet, and an engaging portion is provided in the cover portion, keeping the lancet at the standby position against the biasing force of the spring assembly based on an action of engaging with the housing where the tip portion of the piercing body is arranged within the housing under the condition where the lancet is biased by the spring assembly in the protruding direction of the piercing body.
